# EUROPEAN PATENT SPECIFICATION

(11) **EP 3 541 185 B1**
(45) Date of publication and mention of the grant of the patent: **24.04.2024**
(21) Application number: 17871457.2
(22) Date of filing: 15.11.2017
(51) Int. Cl.: A61K 31/137, A61K 31/197, A61K 31/198, A61K 31/44, A61K 45/06, A61P 3/00, A61P 9/12, A61P 11/00, A61P 43/00

(54) **USE OF 2-HYDROXYBENZYLAMINE IN THE TREATMENT AND PREVENTION OF PULMONARY HYPERTENSION**
VERWENDUNG VON 2-HYDROXYBENZYLAMIN ZUR BEHANDLUNG UND VORBEUGUNG VON PULMONALER HYPERTONIE
UTILISATION DE 2-HYDROXYBENZYLAMINE DANS LE TRAITEMENT ET LA PRÉVENTION DE L'HYPERTENSION PULMONAIRE

(30) Priority: 15.11.2016 US 201662422486 P
(43) Date of publication of application: 25.09.2019
(73) Proprietor: Vanderbilt University, Nashville, TN 37240 (US)
(72) Inventor: FESSEL, Joshua, P., Nashville, TN 37205 (US); ROBERTS, L., Jackson, Gallatin, TN 37066 (US); WEST, James, Nashville, TN 37221 (US)
(74) Representative: Mewburn Ellis LLP
(86) International application number: PCT/US2017/061854
(87) International publication number: WO 2018/093936

(56) References cited:
- WO-A1-2015/127163
- US-A1- 2012 157 501
- US-A1- 2015 031 068
- US-A1- 2016 199 463
- US-B2- 8 709 434
- ROBERT A. EGNATCHIK ET AL: "Dysfunctional BMPR2 signaling drives an abnormal endothelial requirement for glutamine in pulmonary arterial hypertension", PULMONARY CIRCULATION, vol. 7, no. 1, 1 January 2017 (2017-01-01) , pages 186-199, XP055695668, US ISSN: 2045-8932, DOI: 10.1086/690236
- LANE ET AL.: 'Oxidative injury is a common consequence of BMPR2 mutations' PULMONARY CIRCULATION vol. 1, no. 1, 2011, pages 72 - 83, XP055485610
- PAULIN ET AL.: 'Sirtuin 3 Deficiency Is Associated with Inhibited Mitochondrial Function and Pulmonary Arterial Hypertension in Rodents and Humans' CELL METABOLISM vol. 20, 2014, pages 827 - 839, XP055485614

## Description

### GOVERNMENT SUPPORT

This invention was made with government support under grant numbers K08 HL121174, K23 HL098743, R01 HL095797, P01 HL108800, T32 HL007106-34, T32 HL-007891 (identify the contract) awarded by the National Institutes of Health. The government has certain rights in the invention.

### BACKGROUND AND SUMMARY OF THE INVENTION

Pulmonary arterial hypertension (PAH) is increasingly recognized as a systemic disease driven by alteration in the normal functioning of multiple metabolic pathways affecting all of the major carbon substrates, including amino acids. The present inventors found that human pulmonary hypertension patients (WHO Group I, PAH) exhibit systemic and pulmonary-specific alterations in glutamine metabolism, with the diseased pulmonary vasculature taking up significantly more glutamine than that of controls. Using cell culture models and transgenic mice expressing PAH-causing BMPR2 mutations, the present inventors found that the pulmonary endothelium in PAH shunts significantly more glutamine carbon into the tricarboxylic acid (TCA) cycle than wild-type endothelium. Increased glutamine metabolism through the TCA cycle is required by the endothelium in PAH to survive, to sustain normal energetics and to manifest the hyperproliferative phenotype characteristic of disease. The strict requirement for glutamine is driven by loss of sirtuin-3 (SIRT3) activity through covalent modification by reactive products of lipid peroxidation.

Compounds for use according to the present invention, including 2-hydroxybenzylamine (alternatively named salicylamine, SAM, or 2HOBA), scavengers of reactive lipid peroxidation products, have an effect that SIRT3 function is preserved, glutamine metabolism is normalized, and the development of PAH in BMPR2 mutant mice is prevented. In PAH, targeting glutamine metabolism and the mechanisms that underlie glutamine-driven metabolic reprogramming represent a viable novel avenue for the development of PAH therapeutics.

Alterations in the normal metabolic strategies utilized by various cell types are increasingly recognized as part of a central pathogenic mechanism in pulmonary arterial hypertension.^{1,2} Any given cell type - endothelium, smooth muscle, myocardium, skeletal muscle, etc. - exhibits a "metabolic program" under healthy, homeostatic conditions that is the sum total of the use and fate of all of the available carbon sources (primarily carbohydrates, fats, and amino acids). The details of a cell's metabolic program are often particular for that cell type. For example, under normal conditions, cardiac myocytes primarily oxidize fatty acids as an energy source, whereas endothelial cells preferentially use glucose through oxidative and non-oxidative pathways.^{3,4} Any perturbation that places demands upon a cell to increase energy production, to increase macromolecule synthesis, or to resist pro-death stimuli will place a strain on the cell's carbon resources and necessarily change the cell's metabolic program. Conversely, anything that restricts a cell's ability to use one or more carbon substrates can induce a metabolic reprogramming that will often change one or more fundamental properties of the cell, such as differentiation state, proliferative rate, or sensitivity to apoptosis. Thus, a cell's metabolic program is inextricably linked to the role that cell plays in health and disease.

In PAH, it is well recognized that multiple cell types involved in disease pathogenesis exhibit a metabolic reprogramming characterized by increased shunting of glucose-derived carbon into non-oxidative, lactate-producing pathways in spite of the presence of ample oxygen supply to permit oxidative glucose metabolism.^{1,2,5-7} This is colloquially referred to as the "Warburg effect," first described by Otto Warburg as a feature of cancer cells. However, the network of metabolic pathways within a cell is highly interconnected, and it is rare for one pathway to be altered in isolation. Indeed, it is increasingly recognized that fatty acid metabolism is also markedly altered in PAH, and that the reciprocal relationship between glucose and fatty acid oxidation (the so-called "Randle cycle") is abnormal in PAH and likely contributes to pathogenesis in both the heart and in the pulmonary vasculature.⁸⁻¹³ The third major cellular carbon source - amino acids generally, and glutamine specifically - remains relatively understudied in PAH.¹⁴ Though amino acids represent the third major carbon source used by most cells, amino acid trafficking has mainly been studied in PAH in the context of nitric oxide synthesis. Recent discoveries in cancer biology have placed amino acids generally, and glutamine specifically, in central roles for biosynthesis, cellular energetics, and redox homeostasis.¹⁵⁻¹⁸ The present inventors examined glutamine metabolism in PAH in the specific context of dysfunctional signaling through bone morphogenic protein receptor type 2 (BMPR2), which showed that the pulmonary endothelium in PAH would exhibit an abnormal increase in glutamine metabolism as a primary carbon source, in a manner similar to what has been observed in cancer.

Emerging data suggests that the majority of WHO Group I pulmonary hypertension is driven by dysfunction of BMP signaling, specifically dysfunctional BMPR2 signaling. The present inventors have focused on loss-of-function genetic mutations in BMPR2 associated with heritable PAH, but the molecular consequences of impaired BMPR2 signaling are similar across the known variants of WHO Group I disease.

Thus, the present invention is directed to compounds, including salicylamine, for use in the prevention or treatment of cases of WHO Group I pulmonary vascular disease.

In the United States, WHO Group II pulmonary hypertension is the most common form of pulmonary hypertension overall. This type of PH has been linked to the metabolic syndrome and to oxidative stress. More recently, loss of function of SIRT3 has been implicated as a key molecular mechanism driving the development of WHO Group II PH. Given our finding that salicylamine works in multiple contexts by preserving the activity of sirtuin isoforms (and SIRT3 in particular), the present inventors presume that salicylamine would show efficacy in treating or preventing WHO Group II pulmonary hypertension.

WHO Group III pulmonary vascular disease is linked to diseases of the lung that result in chronic or intermittent hypoxia. This is a stimulus known to drive overproduction of reactive oxygen species, activation of pathways that require loss of SIRT3 function, metabolic reprogramming, and structural remodeling of vessels including fibrosis. All of these pathogenic processes have been discovered to be at least partly ameliorated by compounds of the present invention, particularly salicylamine.

WHO Group IV: Chronic thromboembolic pulmonary hypertension (CTEPH) is an uncommon but devastating complication of venous thromboembolism/pulmonary embolism. This is typically a disease involving failure of clot resolution as opposed to ongoing overproduction of new blood clots. Though the underlying biology is still being worked out, oxidative stress and metabolic alterations have been implicated in the pathogenesis of CTEPH.

WHO Group V: This is a category of pulmonary vascular disease with no obvious unifying pathogenic mechanism. However, many of the constituent associated diseases in Group V, such as sarcoidosis, chronic kidney disease, thyroid disease, systemic metabolic disorders, and autoimmune vasculitides, all have oxidative stress as a common pathogenic process.

WO 2015/127163 A1 describes the use of pyridoxamine in the treatment of sickle cell disease, thalassemia and other blood diseases.

US 2012/0157501 A1 describes the use of isoketal scavengers and the mitigation of disorders involving oxidative injury.

Accordingly, disclosed are compounds for use in a method of treating or preventing WHO group I pulmonary hypertension according to claims 1-3.

Additional advantages of the invention will be set forth in part of the description which follows, and in part will be obvious from the description, or can be learned by practice of the invention. The advantages of the invention will be realized and attained by means of the elements and combinations particularly pointed out in the appended claims. It is to be understood this description is exemplary and not restrictive of the invention as claimed. However, the invention is defined by the claims.

### BRIEF DESCRIPTION OF THE FIGURES

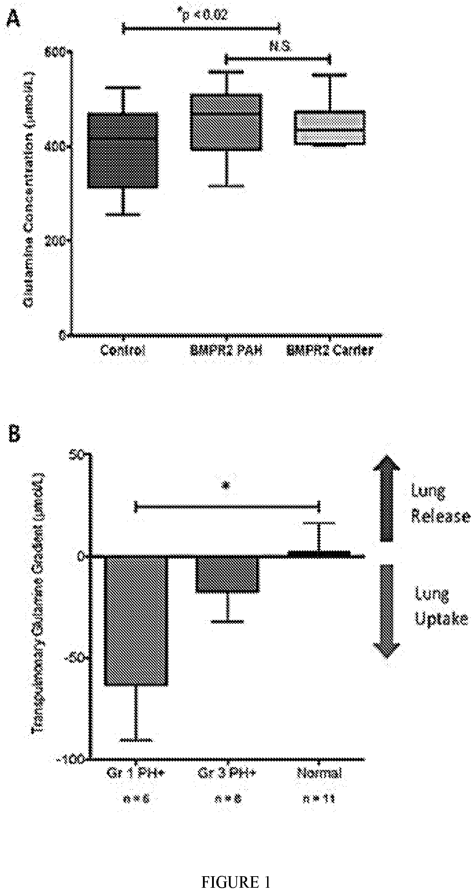

Figure 1 is a graph that shows humans with PAH and impaired BMPR2 signaling exhibit systemic and pulmonary vascular reprogramming of glutamine metabolism. A) Humans with BMPR2 mutations, irrespective of the presence or absence of diagnosed PAH, have a statistically significant increase in circulating glutamine compared to family members with WT BMPR2. N = 11-24, *p < 0.02. B) Transpulmonary glutamine uptake measured at right heart catheterization is significantly increased in patients with WHO Group I PAH compared to individuals with normal hemodynamics and to individuals with WHO Group III pulmonary hypertension. N = 6-11, *p < 0.05.

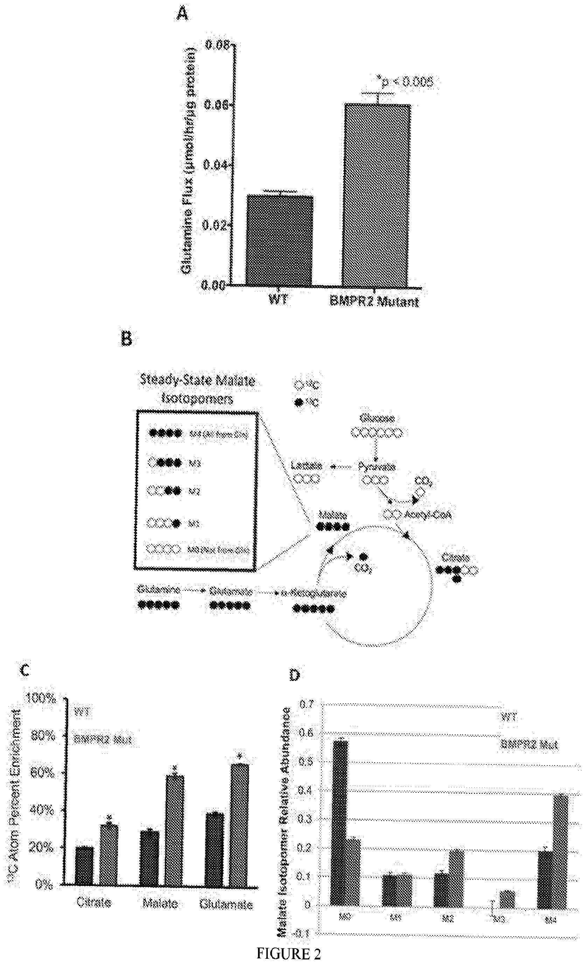

Figure 2 shows BMPR2 mutant PMVECs shuttle significantly more glutamine into the TCA cycle. A) Specific extracellular glutamine uptake flux rates for BMPR2 mutant PMVECs are double those measured for WT PMVECs. N = 4 independent experiments, *p < 0.005 by two-tailed t-test. B) Concept schematic for [U-¹³C₅]-L-glutamine labeling to determine carbon flow into the TCA cycle. C) Compared to WT, BMPR2 mutant PMVECs approximately double the amount of glutamine-derived carbon that is incorporated into multiple TCA cycle intermediates (citrate, malate, and glutamate, the immediate precursor for α-ketoglutarate) by ¹³C %atom enrichment quantified by mass spectrometry. N=4, *p < 0.05 by two-way ANOVA with Tukey post-hoc test. D) ¹³C₅-L-glutamine labeling and quantification of TCA cycle intermediates by mass spectrometry shows a marked shift in the intracellular fate of glutamine-derived carbon in BMPR2 mutant PMVECs compared to WT, exemplified by the isotopomers of malate. In WT cells, the majority of TCA cycle carbon is unlabeled, represented by the M0 isotopomer, indicating use of an unlabeled carbon source (primarily glucose). By contrast, >60% of the TCA carbon in the BMPR2 mutant PMVECs shows incorporation of at least 2 labeled carbons, with 40% of the total malate pool being fully labeled, represented by the M4 isotopomer. N = 3 each for WT and BMPR2 mutant.

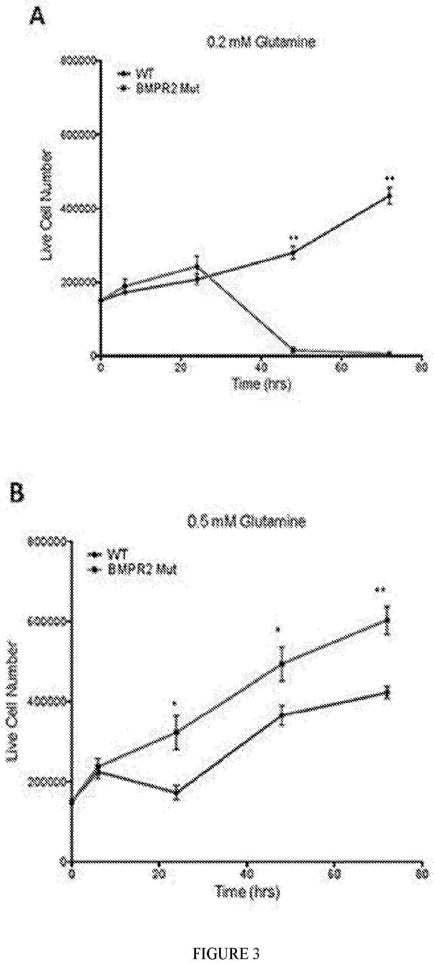

Figure 3 shows BMPR2 mutant PMVEC require increased glutamine availability to manifest hyperproliferative behavior typical of PAH. A) In glutamine-limited conditions (0.2 mM), WT PMVEC are able to maintain steady proliferation, but BMPR2^{R899X} PMVEC begin to die rapidly after 24 hours in culture. B) In more glutamine-replete conditions (0.5 mM), WT PMVEC are essentially unaffected, whereas BMPR2^{R899X} PMVEC exhibit the hyperproliferative phenotype characteristic of PAH. N=6 for each timepoint, *p < 0.01, **p < 0.001 by two-sided t-test.

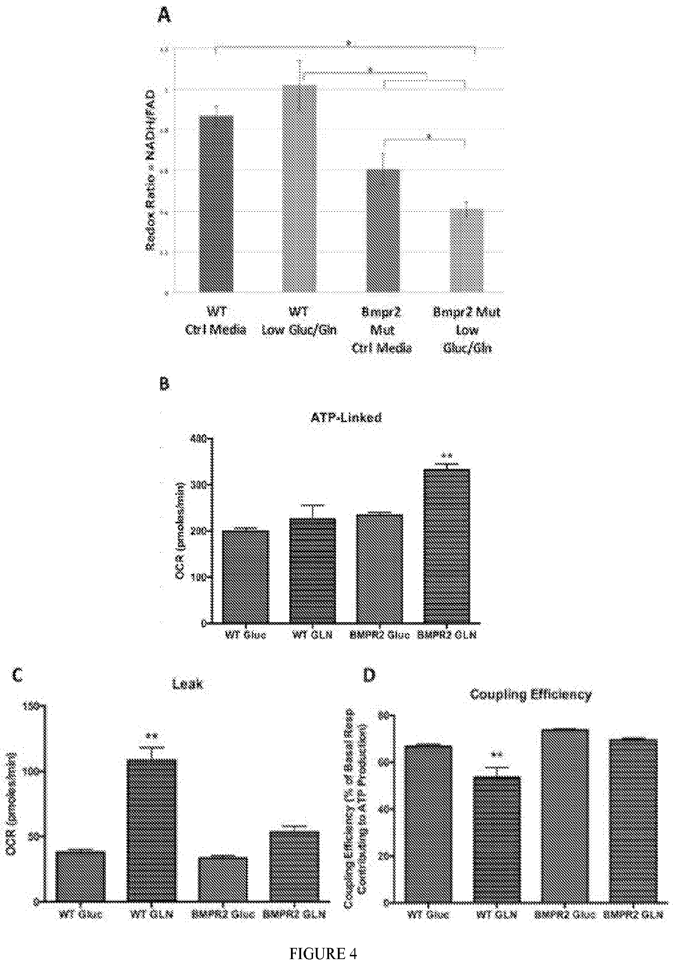

Figure 4 shows PAH-causing BMPR2 mutations drive mitochondrial dysfunction and metabolic reprogramming toward glutamine preference. A) Quantifying intracellular NADH and FAD+ pools in live cells using two-photon autofluorescence to calculate the optical redox ratio showed WT cultured human PMVECs retain metabolic flexibility by maintaining intracellular redox status despite limitation of the availability of glucose and glutamine as carbon sources. By contrast, PMVECs expressing mutant BMPR2 exhibit substantial reduction of redox ratio when glucose and glutamine are limited. N=3 in standard media, N=6 in glucose-free/glutamine-free media, *p<0.05 by two-way ANOVA with Tukey post-hoc testing. B-D) Compared to WT, cultured murine PMVECs expressing mutant Bmpr2 exhibit enhanced glutamine-supported ATP-linked mitochondrial respiration (panel B, **p < 0.0001 by ANOVA vs. all other groups), decreased leak respiration (panel C, **p < 0.0001 by ANOVA vs. all other groups), and increased coupling efficiency (panel D, **p < 0.0001 by ANOVA vs. all other groups). N = 8-10 measurements for each condition per experiment, experiments performed in duplicate.

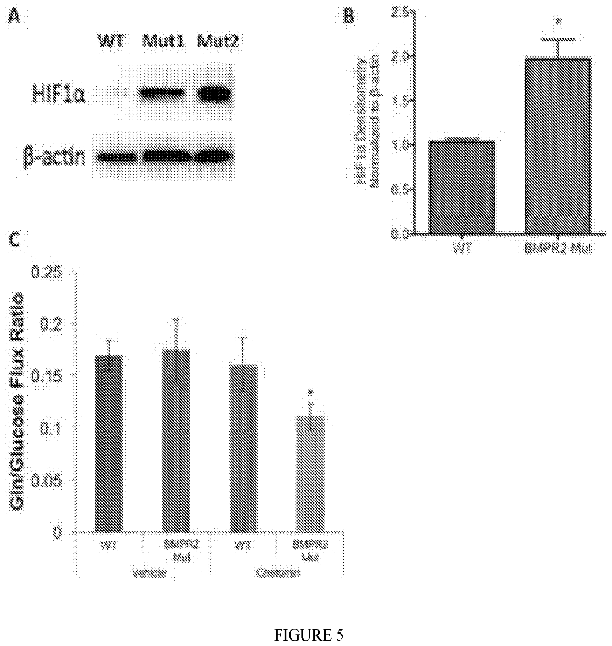

Figure 5 shows Normoxic HIF1α activation in BMPR2 mutant PMVEC contributes to metabolic reprogramming. A-B) HIF1α is increased under normoxic conditions in two different types of BMPR2 mutations expressed in PMVEC (A) and is quantified by densitometry (B), N = 3 independent experiments each for human and murine PMVEC, data analyzed in aggregate with *p < 0.05 by two-tailed t-test. C) Treatment of BMPR2 mutant PMVEC with low-dose chetomin, a pharmacologic inhibitor of HIF1α, significantly reduced the glutamine to glucose flux ratio while leaving WT PMVEC essentially unaffected. N = 3 for each condition, *p < 0.05.

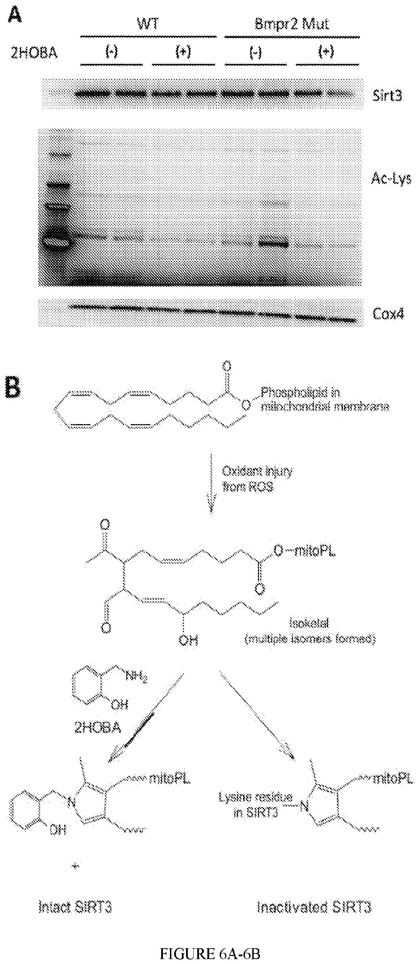

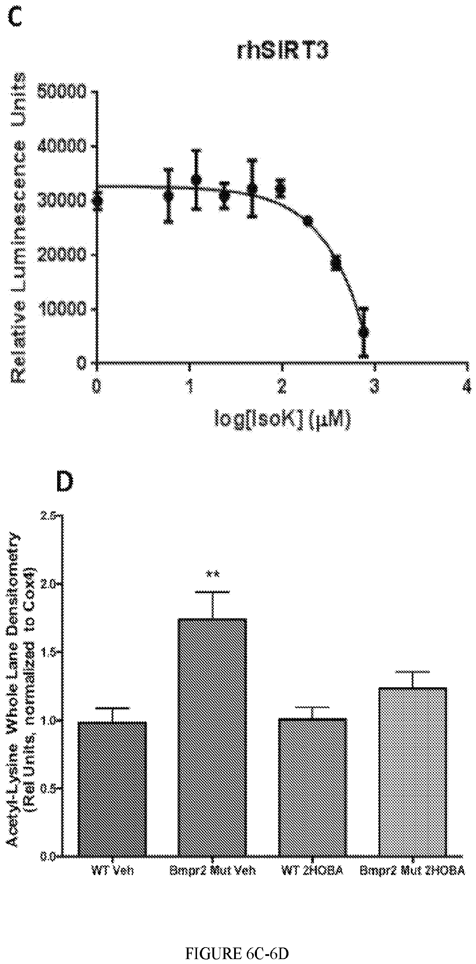

Figure 6 shows SIRT3 is inactivated in BMPR2^{R899X} mice. A) Mitochondria isolated from BMPR2^{R899X} mice have equivalent SIRT3 content compared to WT, but exhibit hyperacetylation of multiple mitochondrial proteins (lanes 1-2 and 5-6), indicating loss of SIRT3 function. Treatment of BMPR2^{R899X} mice with 2-hydroxybenzylamine (2HOBA), a scavenger of isoketals, normalized mitochondrial protein acetylation state (lanes 3-4 and 7-8). Western images are separate serial exposures of the same blot following stripping and reprobing with antibodies to the indicated proteins. B) Schematic showing how oxidative injury to the mitochondrial membrane is thought to inactivate SIRT3 via adduction by isoketals. C) Purified recombinant human SIRT3 activity measured by luminescence assay (Sirt-Glo, Promega) is dose-dependently inhibited by isoketals. D) Densitometry quantification of 4-9 animals from experiments performed in triplicate. **p < 0.01 vs. all other groups by ANOVA with Tukey post-hoc testing.

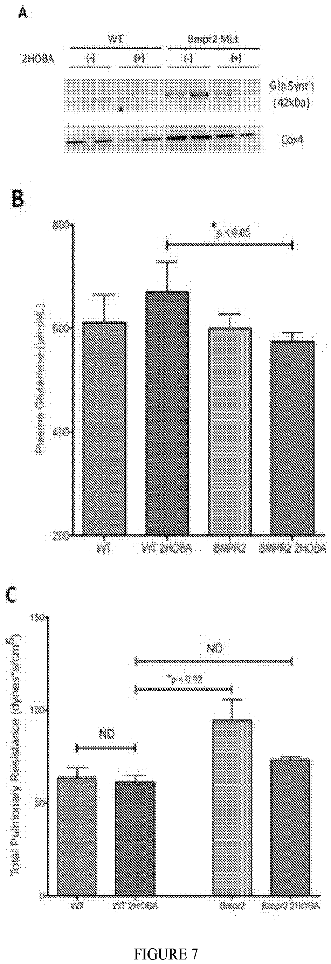

Figure 7 shows 2HOBA lowers circulating glutamine and prevents the development of PAH in BMPR2^{R899X} mice. A) Glutamine synthetase in the liver mitochondrial fraction is elevated in BMPR2^{R899X} mice (lanes 5-6) compared to WT (lanes 1-2). 2HOBA treatment reduced glutamine synthetase in the BMPR2^{R899X} mice (lanes 7-8) but not substantially in WT (lanes 3-4). Western images are separate serial exposures of the same blot following stripping and reprobing with antibodies to the indicated proteins. B) Treatment with 2HOBA significantly reduces plasma glutamine availability in BMPR2^{R899X} mice compared to 2HOBA-treated WT mice. N = 6-29, *p < 0.05. C) 2HOBA treatment reduced total pulmonary resistance in BMPR2^{R899X} mice to a level statistically indistinguishable from WT. N = 5-14, *p < 0.02 by ANOVA with Tukey post-hoc analysis.

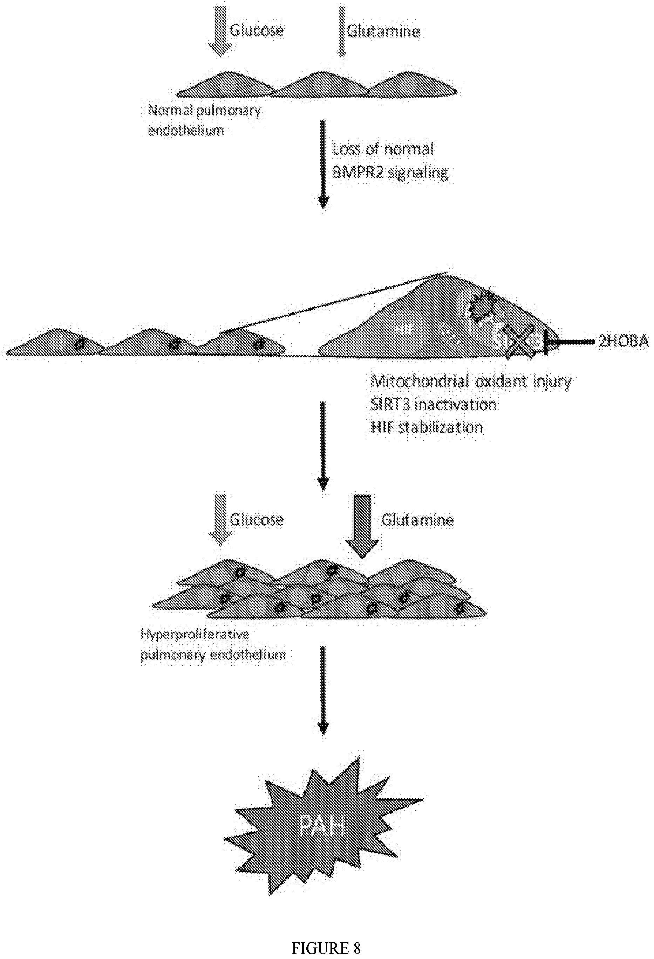

Figure 8 shows a schematic of BMPR2-Mediated Metabolic Reprogramming. The normal pulmonary endothelium relies mainly on glucose as its bioenergetic fuel. When normal BMPR2 signaling is lost, oxidant injury in the mitochondria drives inactivation of SIRT3 via adduction by isoketals. This can be interrupted with 2-hydroxybenzylamine. Unchecked, however, continued oxidant injury and SIRT3 inactivation lead to HIF stabilization, all of which drives a hyperproliferative, glutamine avid pulmonary endothelial phenotype that underlies the development of PAH.

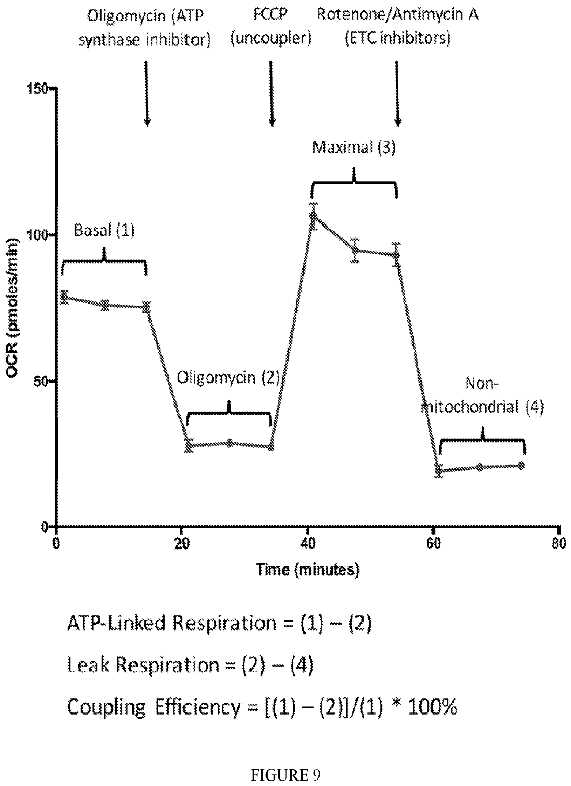

Figure 9 shows a schematic of measurement of mitochondrial respiration in the Seahorse Extracellular Flux analyzer and calculation of respiration parameters. The blue curve is representative data of the oxygen consumption rate (OCR, pmol/min) over time as measured in cultured PMVEC. Specific portions of the curve representing basal respiration (1), oligomycin-sensitive respiration (2), maximal respiration (3), and non-mitochondrial respiration (4) are achieved with addition of the indicated compounds (oligomycin A, FCCP, antimycin A/rotenone). ATP-linked respiration, leak respiration, and coupling efficiency are each calculated utilizing the various parts of the respiration profile as shown.

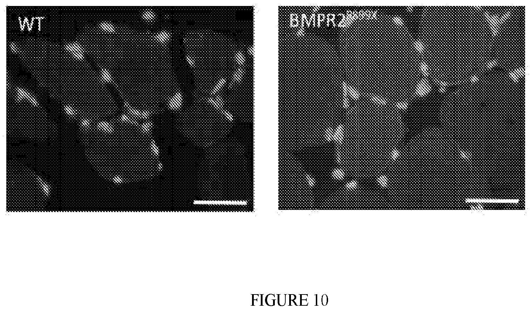

Figure 10 shows glutamine synthetase in skeletal muscle is upregulated in BMPR2^{R899X} mice. Gastrocnemius muscle was fixed and stained with antibody to glutamine synthetase (red) and counterstained with DAPI nuclear stain (blue). Expression of the BMPR2^{R899X} mutation upregulates glutamine synthetase in skeletal muscle, one of the major sites of glutamine synthesis *in vivo,* compared to WT muscle. Scale bar = 25 microns.

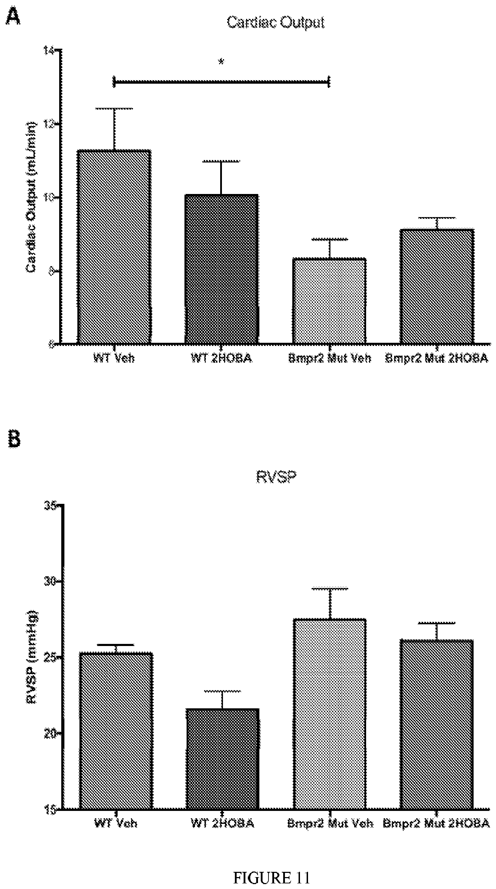

Figure 11 shows that 2HOBA improves both cardiac output and right ventricular systolic pressure (RVSP) in BMPR2^{R899X} mice. Expression of the BMPR2R899X mutation significantly reduces cardiac output (A) and modestly raises RVSP (B) compared to WT. Cardiac output trends toward improved, and RVSP trends toward a decrease in BMPR2^{R899X} treated with 2HOBA compared to vehicle-treated mutants, though neither reaches the values measured in 2HOBA-treated WT mice.

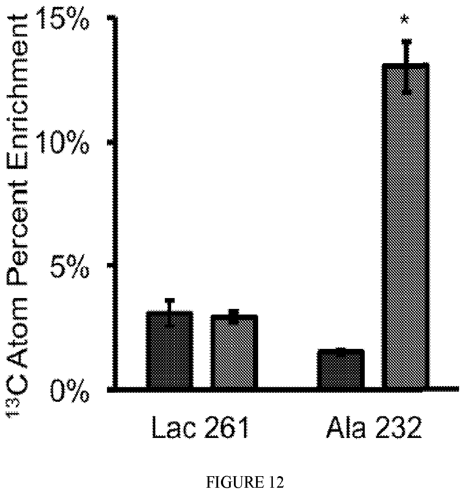

Figure 12 shows that glutamine-derived carbon is disproportionately incorporated into the alanine pool in BMPR2 mutant PMVEC. The intracellular lactate pool shows a small amount of ¹³C incorporation, quantified by mass spectrometry, from labeled glutamine and does not differ between WT and BMPR2 mutant PMVECs. By contrast, there is substantial ¹³C enrichment from labeled glutamine in the alanine pool in BMPR2 mutant PMVECs compared to WT, indicating that the increased glutamine-derived carbon in BMPR2 mutant cells does not contribute to metabolic pathways upstream from pyruvate and the TCA cycle, but likely does contribute to pyruvate cycling. N = 3, *p < 0.05.

### DESCRIPTION OF THE INVENTION

The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which need to be independently confirmed.

As used in the specification and the appended claims, the singular forms "a," "an" and "the" include plural referents unless the context clearly dictates otherwise. Thus, for example, reference to "a functional group," "an alkyl," or "a residue" includes mixtures of two or more such functional groups, alkyls, or residues, and the like.

Ranges can be expressed herein as from "about" one particular value, and/or to "about" another particular value. When such a range is expressed, a further aspect includes from the one particular value and/or to the other particular value. Similarly, when values are expressed as approximations, by use of the antecedent "about," it will be understood that the particular value forms a further aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" means that the subsequently described event or circumstance can or can not occur, and that the description includes instances where said event or circumstance occurs and instances where it does not.

As used herein, the term "subject" refers to a target of administration. The subject of the herein disclosed methods can be a vertebrate, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. A patient refers to a subject afflicted with a disease or disorder. The term "patient" includes human and veterinary subjects.

As used herein, the term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed. As can be seen herein, there is overlap in the definition of treating and preventing.

As used herein, the term "diagnosed" means having been subjected to a physical examination by a person of skill, for example, a physician, and found to have a condition that can be diagnosed or treated by the compounds, compositions, or methods disclosed herein. As used herein, the phrase "identified to be in need of treatment for a disorder," or the like, refers to selection of a subject based upon need for treatment of the disorder. For example, a subject can be identified as having a need for treatment of a disorder (e.g., a disorder related to inflammation) based upon an earlier diagnosis by a person of skill and thereafter subjected to treatment for the disorder. It is contemplated that the identification can, in one aspect, be performed by a person different from the person making the diagnosis. It is also contemplated, in a further aspect, that the administration can be performed by one who subsequently performed the administration.

As used herein, the terms "administering" and "administration" refer to any method of providing a pharmaceutical preparation to a subject. Such methods are well known to those skilled in the art and include, but are not limited to, oral administration, transdermal administration, administration by inhalation, nasal administration, topical administration, intravaginal administration, ophthalmic administration, intraaural administration, intracerebral administration, rectal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the term "effective amount" refers to an amount that is sufficient to achieve the desired result or to have an effect on an undesired condition. For example, a "therapeutically effective amount" refers to an amount that is sufficient to achieve the desired therapeutic result or to have an effect on undesired symptoms, but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products. In further various aspects, a preparation can be administered in a "prophylactically effective amount"; that is, an amount effective for prevention of a disease or condition.

As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose. Desirably, at least 95% by weight of the particles of the active ingredient have an effective particle size in the range of 0.01 to 10 micrometers.

As used herein, the term "scavenger" or "scavenging" refers to a chemical substance that can be administered in order to remove or inactivate impurities or unwanted reaction products. For example, the isoketals irreversibly adduct specifically to lysine residues on proteins. The isoketal scavengers of the present invention react with isoketals before they adduct to the lysine residues. Accordingly, the compounds of the present invention "scavenge" isoketals, thereby preventing them from adducting to proteins.

Compounds for use according to the present invention rapidly bind γ-KAs to "scavenge" these injurious mediators to prevent oxidative protein modification, as an alternative approach to upstream therapy. One of the compounds of the present invention, salicylamine, is a natural product with an excellent safety profile in pre-clinical animal studies. Moreover, salicylamine prevents the formation of both γ-KAs and toxic protein oligomers with remarkable therapeutic benefit in animal models of Alzheimer's disease and hypertension. The present inventors have identified protein oligomers and oxidative stress/formation of γ-KAs in cellular and *in vivo* models associated with PAH susceptibility. Importantly, the present inventors have demonstrated a beneficial effect of scavenging γ-KAs to modulate glutamine metabolism and increase the activity of SIRT3. Therefore, the compounds of the present invention, including salicylamine, represent a completely novel therapy to prevent and treat pulmonary hypertension.

In a preferred embodiment, the compound for use is salicylamine (2-hydroxybenzylamine or 2-HOBA).

The compound may be chosen from: 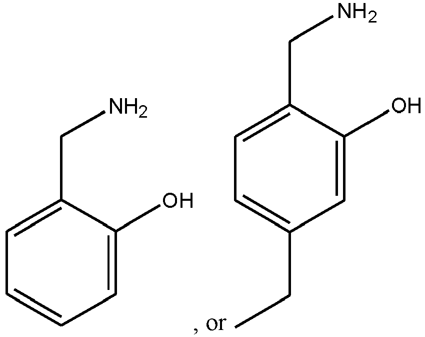 or a pharmaceutically acceptable salt thereof.

As used herein, the term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (-ic and -ous), ferric, ferrous, lithium, magnesium, manganese (-ic and -ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other embodiments of the present invention include pharmaceutical compositions that may be used in connection with the methods disclosed herein. These compositions include at least one compound for use according to the present invention and a pharmaceutically acceptable carrier. That is, a pharmaceutical composition can be provided comprising a therapeutically effective amount of at least one disclosed compound or at least one product of a disclosed method and a pharmaceutically acceptable carrier.

In certain aspects, the disclosed pharmaceutical compositions comprise the disclosed compounds (including pharmaceutically acceptable salt(s) thereof) as an active ingredient, a pharmaceutically acceptable carrier, and, optionally, other therapeutic ingredients or adjuvants. The instant compositions include those suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions can be conveniently presented in unit dosage form and prepared by any of the methods well known in the art of pharmacy.

In practice, the compounds for use according to the invention, or pharmaceutically acceptable salts thereof, of this invention can be combined as the active ingredient in intimate admixture with a pharmaceutical carrier according to conventional pharmaceutical compounding techniques. The carrier can take a wide variety of forms depending on the form of preparation desired for administration, e.g., oral or parenteral (including intravenous). Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion or as a water-in-oil liquid emulsion. In addition to the common dosage forms set out above, the compounds of the invention, and/or pharmaceutically acceptable salt(s) thereof, can also be administered by controlled release means and/or delivery devices. The compositions can be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions can include a pharmaceutically acceptable carrier and a compound or a pharmaceutically acceptable salt of the compounds of the invention. The compounds for use according to the invention, or pharmaceutically acceptable salts thereof, can also be included in pharmaceutical compositions in combination with one or more other therapeutically active compounds. The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media can be employed. For example, water, glycols, oils, alcohols, flavoring agents, preservatives, coloring agents and the like can be used to form oral liquid preparations such as suspensions, elixirs and solutions; while carriers such as starches, sugars, microcrystalline cellulose, diluents, granulating agents, lubricants, binders, disintegrating agents, and the like can be used to form oral solid preparations such as powders, capsules and tablets. Because of their ease of administration, tablets and capsules are the preferred oral dosage units whereby solid pharmaceutical carriers are employed. Optionally, tablets can be coated by standard aqueous or nonaqueous techniques

A tablet containing the composition of this invention can be prepared by compression or molding, optionally with one or more accessory ingredients or adjuvants. Compressed tablets can be prepared by compressing, in a suitable machine, the active ingredient in a free-flowing form such as powder or granules, optionally mixed with a binder, lubricant, inert diluent, surface active or dispersing agent. Molded tablets can be made by molding in a suitable machine, a mixture of the powdered compound moistened with an inert liquid diluent.

The pharmaceutical compositions can comprise a compound of the invention (or pharmaceutically acceptable salts thereof) as an active ingredient, a pharmaceutically acceptable carrier, and optionally one or more additional therapeutic agents or adjuvants. The instant compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions can be conveniently presented in unit dosage form and prepared by any of the methods well known in the art of pharmacy.

Pharmaceutical compositions suitable for parenteral administration can be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions can be in a form suitable for topical use such as, for example, an aerosol, cream, ointment, lotion, dusting powder, mouth washes, gargles, and the like. Further, the compositions can be in a form suitable for use in transdermal devices. These formulations can be prepared, utilizing a compound of the invention, or pharmaceutically acceptable salts thereof, via conventional processing methods. As an example, a cream or ointment is prepared by mixing hydrophilic material and water, together with about 5 wt% to about 10 wt% of the compound, to produce a cream or ointment having a desired consistency.

Pharmaceutical compositions can be in a form suitable for rectal administration wherein the carrier is a solid. It is preferable that the mixture forms unit dose suppositories. Suitable carriers include cocoa butter and other materials commonly used in the art. The suppositories can be conveniently formed by first admixing the composition with the softened or melted carrier(s) followed by chilling and shaping in molds.

In addition to the aforementioned carrier ingredients, the pharmaceutical formulations described above can include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of the invention, and/or pharmaceutically acceptable salts thereof, can also be prepared in powder or liquid concentrate form.

### EXAMPLES

This Example details research in connection with an embodiment of the present invention.

### Materials and Methods

*Reagents:* ¹³C₅-L-glutamine was purchased from Sigma-Aldrich (St. Louis, MO). Chetomin was purchased from Cayman Chemical (Ann Arbor, MI). 2-Hydroxybenzylamine (2HOBA) was synthesized at Vanderbilt as previously described.¹⁹ Antibodies were purchased as follows: HIF1α and glutamine synthetase, Novus Biologicals (Littleton, CO); Sirt3, Cell Signaling Technology (Danvers, MA); acetyl-lysine, EMD Millipore (Billerica, MA); Cox4, Abcam (Cambridge, MA). Recombinant human SIRT3 was purchased from R&D Systems (Minneapolis, MN). Sirt-Glo assay kit was purchased from Promega (Madison, WI) and used according to manufacturer's instructions.

*Cell Culture:* The present inventors used previously characterized WT and BMPR2 mutant (BMPR2^{R899X}) pulmonary microvascular endothelial cells isolated from conditionally immortalized murine lines generated on the ImmortoMouse background,²⁰ and WT and BMPR2 mutant PMVECs from a parent immortalized human line stably expressing either a WT or BMPR2 mutant construct transfected into the parent line and maintained under selection with G418S. For murine lines, cells were reverted to a primary endothelial phenotype by removal of murine interferon-gamma and transition to 37C (from 33C, which maintains conditional immortal phenotype through expression of SV40 large T), and doxycycline (300 ng/mL) was added to the media to induce expression of the transgene (the construct being Rosa26-rtTA x TetO₇-Bmpr2^{R899X}, the same as that used in Animal Studies), both for at least 72 hr prior to experiments. Human lines stably express WT or mutant BMPR2, and selection with G418S was discontinued for 24 hr prior to experiments.

*Metabolic Fluxes and Stable Isotope Labeling:* Glucose and lactate levels were measured using the YSI 2300 Stat Glucose and Lactate Analyzer (Yellow Springs, OH). High performance liquid chromatography (HPLC) was used to quantify amino acid concentrations, with norvaline as an internal standard. Amino acid samples were then injected onto a Zorbax Eclipse Plus C18 column (Agilent) using a two phase chromatography method as previously described.²¹ For isotope tracer studies, WT and BMPR2 mutant PMVEC were cultured in media containing 2 mM [U-¹³C₅]-glutamine in place of unlabeled glutamine for 24hrs. Analytes were extracted into ice-cold methanol and separated in 1:1 chloroform:H2O. The aqueous phase containing the amino and organic acids was then dried under air at room temperature. The samples were derivatized using MBTSTFA + 1% TBDMCS (Pierce). 2 µL of each derivatized sample was then injected onto 30m DB-35ms capillary column in an Agilent 6890N/5975B GC-MS. Flux rates were calculated using the ETA software package.²²

*Cell Proliferation Assays:* Cells were seeded and grown under specified media conditions as outlined in Results. Cells were counted for total and live cells using Trypan blue exclusion, and automated counts were done using a Countess cell counter (Life Techologies, Grand Island, NY).

*Two-Photon Autofluorescence Measurement of Optical Redox Ratio:* Two-photon images were acquired and analyzed as described previously.²³ Briefly, cells were plated at a consistent density on glass-bottomed dishes and imaged 48 hours later. A two-photon microscope (Bruker) and 40x oil-immersion objective (1.3NA) were used to acquire NADH and FAD autofluorescence images for the same fields of view. Images were imported into MATLAB (Mathworks), and the NADH intensity was divided by the FAD intensity for each pixel to calculate a redox ratio image. The redox ratio was averaged across each image.

*Mitochondrial Functional Studies:* To assess mitochondrial respiration in cultured PMVECs, cells were seeded in growth media in a 96-well plate from Seahorse Biosciences (Bilerica, MA) at a seeding density of 50,000 live cells per well. The following day, cells were washed and placed in Seahorse Assay Media supplemented with specific substrates (1 gm/L glucose, 2 mM L-glutamine). After equilibration, oxygen consumption rates were measured on a Seahorse XFe96 analyzer using the Mito Stress Test protocol. For mitochondrial protein measurements, mitochondria were isolated from fresh murine liver tissue as previously described.²⁴

*Animal Studies:* All animal studies were approved by the Vanderbilt IACUC. Male and female FVB/N mice were 10-16 weeks of age. BMPR2^{R899X} mice were generated and maintained as previously described.^{24,25} For 2HOBA studies, 1 gm/L was administered in the drinking water, with water bottles protected from light and water changes every 2-3 days.
Echocardiography was performed on anesthetized mice (isoflurane anesthesia) to determine cardiac output. Right ventricular systolic pressure (RVSP) was invasively measured as described, using bromoethanol as the anesthetic.^{24,25}

Human Studies: All human studies were approved by the Institutional Review Boards of Vanderbilt University, the University of North Carolina at Chapel Hill, and Yale University. Informed consent was obtained from each patient. For measurements of circulating glutamine levels, blood samples were obtained by peripheral venipuncture, centrifuged to separate plasma, and stored at -80°C until analysis. Amino acid profiles were quantified in Vanderbilt's clinical chemistry laboratory using a standard CLIA-approved method. For transpulmonary measurements, samples were obtained at the time of clinically indicated diagnostic right heart catheterization as previously described.^{26,27} Patients were classified as having pulmonary hypertension or not based upon the results of invasive hemodynamic testing, with differences between WHO groups determined by clinical history.

*Statistical Analyses:* All statistics were performed using GraphPad Prism 6.0 or Microsoft Excel unless otherwise specified. For animal experiments, sample sizes were chosen based upon previous experiments with approximately 80% of transgenic animals showing RVSP > 30 mm Hg. Animals were randomly assigned to treatment groups by cage. For all measurements, investigators were blind to treatments and genotype until data analysis. Data were analyzed using two-tailed t-test, or two-way ANOVA with Tukey's post-test, as indicated. Significance was set at α < 0.05 after correction for multiple comparisons.

### Results

*Glutamine Metabolism is Abnormal in Human PAH Patients:* To test the hypothesis that PAH involves alteration of glutamine metabolism, the present inventors quantified fasting serum glutamine levels in heritable PAH patients with known BMPR2 mutations, in unaffected mutation carriers (individuals with known BMPR2 mutations but no evidence of PAH), and in married-in controls from the same households as patients and carriers. The present inventors found that circulating glutamine levels were significantly elevated in both heritable PAH patients (451 +/- 68 umol/L) and in BMPR2 mutation carriers (450 +/- 50 umol/L) compared to controls (399 +/- 82 umol/L, p < 0.05, **Figure 1A**). This was unexpected, as previous work has suggested increased cardiac glutamine uptake is a feature of the aberrant metabolic program in PAH.¹⁴ To better quantify glutamine metabolism within the pulmonary vasculature, the present inventors measured transpulmonary glutamine gradients in WHO Group I PAH patients, WHO Group III PH patients, and individuals with normal pulmonary hemodynamics. Samples were collected from the main pulmonary artery and from the pulmonary capillary wedge position at the time of diagnostic right heart catheterization.²⁶ Glutamine concentrations were quantified in each sample, and the difference between the PCW and the PA samples for each individual was the gradient measurement (negative values indicate net uptake, positive values indicate net release). WHO Group I PAH patients showed substantial glutamine uptake by the pulmonary vasculature compared to WHO Group III patients and to controls **(****Figure 1B****).** Taken together, these data indicated that PAH patients with abnormal BMPR2 function have marked changes in whole body and in pulmonary vascular glutamine metabolism.

*BMPR2 Mutant Endothelium Exhibits Abnormal Avidity for Glutamine Carbon:* The present inventors determined whether increased glutamine uptake by pulmonary endothelial cells with dysfunctional BMPR2 is an intrinsic property of those cells or merely due to the increased availability of glutamine. To assess this, wild-type (WT) and BMPR2 mutant pulmonary microvascular endothelial cells (PMVEC) were grown in culture and provided glutamine in significant excess (2 mM) of physiologic levels in the culture media. Under conditions of glutamine excess, BMPR2 mutant PMVEC took up glutamine at twice the rate of WT cells **(****Figure 2A****),** suggesting that increased glutamine uptake is intrinsic to PMVEC with dysfunctional BMPR2 signaling.

Glutamine can be used as a source of carbon input to the TCA cycle, but it is also an important source of nitrogen in the cell, supplying nitrogen-requiring processes such as nucleotide synthesis. The present inventors hypothesized that glutamine was being used as a carbon source, and that it was being preferentially shunted to the TCA cycle in BMPR2 mutant PMVEC compared to WT. To test this hypothesis, WT and BMPR2 mutant PMVEC were cultured for 24 hours in the presence of 2 mM [U-¹³C₅]-L-glutamine, a stable isotope of glutamine in which all 5 carbon atoms are carbon-13, which is easily detectable by mass spectrometry (see schematic **Figure 2B****).** The other major cellular carbon sources (glucose, fatty acids) were left unlabeled, and the serum used had been dialyzed to remove free amino acids. Compared to WT, BMPR2 mutant PMVEC showed excess incorporation of glutamine-derived carbon in multiple intermediates of the TCA cycle **(****Figure 2C****).** Furthermore, when specific TCA intermediates (e.g., malate) were broken down by tandem mass spectrometry to determine how many glutamine-derived carbon atoms had been incorporated (0, 1, 2, 3, or 4 for malate), the present inventors found that the majority of malate present (40%) contained 4 atoms of ¹³C, indicating that the majority of the carbon in the TCA cycle was coming from glutamine, as this was the sole source of ¹³C available **(****Figure 2D****).** By contrast, in WT PMVEC, the majority of the malate present (55+%) contained no atoms of ¹³C, indicating that in WT cells, the majority of the carbon in the TCA cycle was not coming from glutamine but rather from the unlabeled glucose and fatty acids present.

### Glutamine is a Required Carbon Source for BMPR2 Mutant PMVEC

The present inventors next determined whether BMPR2 mutant PMVEC have an absolute requirement for glutamine, or whether this represented an "arrangement of convenience" as a result of the presence of excess glutamine. The present inventors cultured WT and BMPR2 mutant PMVEC in two concentrations of glutamine, 500 µM (mimicking serum concentrations in BMPR2 mutant patients) and 200 µM (representing the lowest levels that would be considered normal in humans). At 500 µM glutamine, BMPR2 mutant PMVEC show a net proliferation that exceeds the rate of WT PMVEC at all timepoints out to 72 hours **(****Figure 3A**). At 200 µM glutamine, the net proliferative rate of WT PMVEC is essentially unchanged, but the BMPR2 mutant PMVEC are completely intolerant of glutamine-limited conditions and have all died by 72 hours **(****Figure 3B****).**

To better understand the details of glutamine utilization and the effects of glutamine limitation in BMPR2 mutant PMVEC, the present inventors quantified total intracellular redox status of WT and BMPR2 mutant PMVEC using two-photon autofluorescence of endogenous NADH and FAD, the major electron carriers controlled by TCA cycle activity. Fluorescence from both species allows calculation of the optical redox ratio, with a higher ratio indicating more TCA cycle activity, a more reduced intracellular redox environment, and increased overall metabolic activity. Conversely, a reduction in the optical redox ratio indicates an overall decrease in metabolic activity, particularly via the TCA cycle.²⁸⁻³⁰ Two-photon autofluorescence has very high time resolution, allowing for rapid changes in the metabolic and redox status of the cell to be detected and quantified. The present inventors quantified the optical redox ratio in WT and BMPR2 mutant PMVEC under basal conditions and with acute withdrawal of glutamine and glucose. At baseline, BMPR2 mutant PMVEC have a lower optical redox ratio than WT cells **(****Figure 4A****),** indicating a relatively impaired ability to maintain the intracellular redox environment and relatively impaired overall metabolic activity. With acute limitation of TCA cycle substrates, WT PMVEC are able to rapidly adapt their metabolic behavior to maintain the optical redox ratio **(****Figure 4A****),** whereas BMPR2 mutant PMVEC show a significant further reduction in the optical redox ratio **(****Figure 4A****),** suggesting a significant loss of acute metabolic flexibility.

Next examined the consequences of TCA cycle glutamine dependence on mitochondrial function in BMPR2 mutant PMVEC. Cultured WT and BMPR2 mutant PMVEC were given either glucose or glutamine as the sole energy substrate available. Mitochondrial oxygen consumption was quantified using the Seahorse XFe96 analyzer, and specific components of cellular respiration (ATP-linked respiration, proton leak, and coupling efficiency) were calculated from the oxygen consumption profiles (see **Figure 9** for schematic). WT PMVEC show equivalent ATP-linked respiration with glucose or glutamine as the sole carbon source, but BMPR2 mutant PMVEC show enhanced ATP-linked respiration with glutamine as their carbon source compared to glucose **(****Figure 4B****).** When using glutamine, WT PMVEC exhibit increased proton leak in the mitochondria compared to when using glucose, but BMPR2 mutant cells maintain low levels of proton leak with either glutamine or glucose **(****Figure 4C****),** suggesting that the overall efficiency for use of glutamine as a fuel to support ATP synthesis is much greater in BMPR2 mutant PMVEC compared to WT. Consistent with this, when comparing ATP-linked respiration to basal respiration, WT endothelial cells exhibited a lower coupling efficiency when using glutamine as fuel, whereas BMPR2 mutant PMVEC maintain a high coupling efficiency **(****Figure 4D****).** Taken together, these data indicated that glutamine is the preferred substrate for energy production in BMPR2 mutant PMVEC.

### Oxidative Stress Drives HIF Activation and SIRT3 Impairment in BMPR2 Mutants

The present inventors next wished to determine the molecular events downstream from BMPR2 mutation that contribute to glutamine reliance. Multiple signaling pathways have been shown to regulate increased glutamine utilization in a variety of disease contexts.^{15,31-35} Of the possible candidate pathways, the pathway with the greatest relevance to PAH that also potently regulates glutamine metabolism is hypoxia-inducible factor 1-alpha (HIF1α). HIF1α has been shown to be stabilized under normoxic conditions in experimental and human PAH, and activation of HIF1α can induce glutamine reliance.³⁶⁻³⁹ The present inventors discovered that BMPR2 mutant PMVEC grown in culture exhibited significant normoxic stabilization of HIF1α at the protein level compared to WT **(****Figure 5A and 5B**), and that this was demonstrable for two different PAH-associated BMPR2 mutation types. Then WT and BMPR2 mutant PMVEC was treated with chetomin, a pharmacologic inhibitor of HIF, and assessed glucose and glutamine uptake by quantifying the extracellular flux ratio of glutamine to glucose. Treatment of WT cells had no effect on the glutamine to glucose flux ratio (**Figure 5C**). However, treating BMPR2 mutant PMVEC with the HIF inhibitor significantly reduced the glutamine to glucose flux ratio **(****Figure 5C****),** suggesting that HIF1 activity helps to drive the glutamine requirement in BMPR2 mutant endothelium.

Though HIF1α activation contributes to glutamine uptake and utilization in BMPR2 mutant cells, the present inventors suspected that there were additional alterations in signaling pathways known to control metabolism. Activation of HIF1α by itself has been shown to drive glutamine metabolism, but the glutamine is disproportionately used for biosynthesis.³⁹⁻⁴¹ Given the finding that glutamine in BMPR2 mutant PMVEC is being used for energy production and not obviously for disproportionate biosynthesis, the present inventors hypothesized that a metabolic control pathway directly involved in both glutamine regulation and energy production was likely altered. Sirtuin-3 (SIRT3), a lysine deacetylase involved in mitochondrial energy production and redox homeostasis, emerged as a strong candidate. Loss of SIRT3 has been shown to lie upstream of HIF1 activation and has been associated with pulmonary hypertension.^{15,42-44}

Because sirtuin expression and activity is tightly regulated by substrate availability, nutrient intake, physical activity, and interacting signaling pathways operating between organ systems, the role of SIRT3 in BMPR2-mediated PAH is best studied in a murine model system. To investigate the role of SIRT3 inactivation in BMPR2-mediated PAH, the present inventors isolated mitochondria from wild-type and BMPR2 mutant (BMPR2^{R899X}) mice fed a Western diet (60% calories from fat) for 8 weeks. The mitochondrial proteome was assessed for acetylation of lysine residues, with SIRT3 inactivation leading to lysine hyperacetylation. Compared to WT, BMPR2^{R899X} mitochondria had equivalent protein levels of SIRT3 but exhibited significant lysine hyperacetylation of multiple mitochondrial proteins **(****Figure 6A****,** lanes 1-2 for WT and 5-6 for mutants, quantified in **Figure 6D****),** consistent with loss of SIRT3 activity in BMPR2^{R899X} mice.

Hyperacetylation of the mitochondrial proteome with no change in SIRT3 protein content is consistent with loss of SIRT3 enzymatic activity. The present inventors have previously shown that the mitochondrial membranes in BMPR2^{R899X} mice exhibit extensive lipid peroxidation, and that the lipid peroxidation is characterized by excess production of covalent adducts between proteins and isoketals, a subset of highly reactive lipid peroxidation products.^{24,45} The present inventors thus hypothesized that SIRT3 was undergoing oxidative inactivation in the mitochondria of BMPR2^{R899X} mice, and that treatment with the isoketal scavenger 2-hydroxybenzylamine (2HOBA) would restore normal SIRT3 activity (schematic in **Figure 6B****).** The present inventors demonstrated the biochemical plausibility of this hypothesis by incubating synthetically pure isoketal with recombinant human SIRT3 *in vitro.* Isoketal treatment inactivated SIRT3 in a concentration-dependent manner as measured by luminescence via the Sirt-Glo assay **(****Figure 6C****).** Then the hypothesis that scavenging isoketals would preserve SIRT3 function by treating WT and BMPR2^{R899X} mice with 1 g/L 2HOBA in their drinking water was tested. Treatment with 2HOBA significantly reduced lysine acetylation in the mitochondrial proteome in BMPR2^{R899X} mice compared to WT without affecting total SIRT3 content in the mitochondria **(****Figure 6A****,** lanes 3-4 for WT and 7-8 for mutants, quantified in **Figure 6D****),** consistent with preservation of SIRT3 catalytic activity.
*2HOBA Normalizes Glutamine Metabolism and Prevents PAH In Vivo:* Having shown that impaired BMPR2 function is associated with loss of SIRT3 function, and that this can be prevented with 2HOBA treatment to scavenge damaging lipid peroxidation products, the present inventors next wished to assess the *in vivo* metabolic dysfunction downstream from BMPR2 mutation, the relationship to PAH, and the effect of 2HOBA treatment. The present inventors hypothesized that 2HOBA would prevent the development of PAH in BMPR2^{R899X} mice and would have a beneficial modulatory effect on glutamine metabolism *in vivo.* Expression of the BMPR2^{R899X} allele was sufficient to drive upregulation of glutamine synthetase in skeletal muscle **(****Figure 10****)** and in liver mitochondria **(****Figure 7A****)** compared to WT. Despite upregulation of glutamine synthetase, circulating glutamine concentrations were equivalent in vehicle-treated WT and BMPR2^{R899X} mice **(****Figure 7B****),** suggesting that increased glutamine synthesis was being matched by increased consumption in the BMPR2 mutants. Following 2HOBA treatment, BMPR2^{R899X} mice had reduced glutamine synthetase in liver mitochondria **(****Figure 7A****)** and significantly lower circulating glutamine compared to treated WT mice **(****Figure 7B****),** suggesting a favorable effect on glutamine balance in the mutants. Consistent with the effects on SIRT3 and glutamine metabolism, 2HOBA treatment prevented the development of pulmonary hypertension as measured by total pulmonary resistance in the BMPR2^{R899X} mice compared to vehicle-treated mice **(****Figure 7C****).** Treatment with 2HOBA in the BMPR2^{R899X} mice modestly increased cardiac output **(****Figure 11A****)** and decreased RVSP **(****Figure 11B****),** the combined effect of which was to significantly reduce total pulmonary resistance.

### Discussion

As part of this example, the present inventors used human and murine cell culture models, transgenic mice, and samples from living PAH patients to demonstrate a markedly altered metabolic program for glutamine due to dysfunctional BMPR2 signaling **(****Figure 8****).** Loss of normal BMPR2 function leads to oxidant injury in the mitochondria and formation of reactive products of lipid peroxidation termed isoketals.⁴⁵ Isoketals inactivate SIRT3 which, together with increased oxidant stress, results in stabilization of HIF1α. Together, SIRT3 and HIF1α are two of the best established "master regulator" pathways for cellular metabolism generally and glutamine metabolism specifically. The present inventors show that this process can be interrupted *in vivo* by treating with an orally bioavailable scavenger of isoketals - 2-hydroxybenzylamine (2HOBA) - and that interruption of the molecular cascade leading to glutamine addiction prevents the development of PAH.

The discovery of the present inventors add to the rapidly growing weight of evidence implicating altered cellular metabolism as a major contributor to the development and maintenance of pulmonary hypertension. The first major metabolic perturbation described in PAH was a shift to lactate-producing glycolysis despite the presence of ample oxygen concentrations to permit complete glucose oxidation. This finding has now been replicated in virtually every setting of experimental and human PAH, and the molecular underpinnings continue to be clarified.^{1,2,6,11,38,46-48} However, as has also been true for cancer research, the present inventors and others are finding that the changes to the cellular metabolic landscape in PAH are not restricted to glycolysis, but rather appear to involve most of the major metabolic pathways. Given the interconnectedness of the metabolome in healthy cells, this is perhaps not surprising. Indeed, the present inventors have previously shown that expression of mutant isoforms of BMPR2 in PMVECs is sufficient to reprogram multiple metabolic pathways.⁴⁷

Our findings help bring together several other recently published reports concerning the altered metabolic program in pulmonary hypertension. Piao et al showed increased RV glutaminolysis in monocrotaline treated rats, and that a signature of increased glutaminolysis was also present in the RV from human PAH patients.¹⁴ Paulin et al recently published that loss of SIRT3 activity through reduction at the protein level predisposed mice to the development of PAH, though the mechanism of downregulation was not entirely clear.⁴⁴ Extending these findings, Lai et al have very recently shown an important role for SIRT3 in pulmonary hypertension and the associated systemic metabolic derangements seen in left heart failure with preserved ejection fraction.⁴⁹ Diebold et al reported metabolic remodeling in pulmonary endothelial cells with reduced BMPR2 expression, though manifestation of the PAH phenotype required exposure to hypoxia, implicating HIF but not SIRT3.^{46,50} Discoveries related to the present invention fill in important gaps within and between these studies by providing a possible mechanism by which all of these published findings could be consistent with one another. In addition, the present inventors provide evidence in living PAH patients that dynamic metabolism is in fact reprogrammed in a manner similar to what has been observed in model systems and post-mortem tissue. The present inventors have also shown that targeting at least one of the mechanisms underlying metabolic reprogramming in PAH has beneficial effects *in vivo,* and the present inventors have done so with a compound that can be used in humans. 2-Hydroxybenzylamine has a very favorable long-term safety profile, with over 12 months of continuous dosing in mice producing no significant toxicity. 2-Hydroxybenzylamine is a naturally occurring product, allowing expedited proof-of-principle studies in humans, with subsequent studies focusing on related compounds with improved pharmacokinetics. Importantly, 2HOBA's mechanism of action has been demonstrated in other disease models and across species.^{19,51}

### References

1. Sutendra G, Michelakis ED. The metabolic basis of pulmonary arterial hypertension. Cell metabolism. 2014;19(4):558-573.
2. Ryan JJ, Archer SL. Emerging concepts in the molecular basis of pulmonary arterial hypertension: part I: metabolic plasticity and mitochondrial dynamics in the pulmonary circulation and right ventricle in pulmonary arterial hypertension. Circulation. 2015;131(19):1691-1702.
3. Taegtmeyer H, Young ME, Lopaschuk GD, Abel ED, Brunengraber H, Darley-Usmar V, Des Rosiers C, et al. Assessing Cardiac Metabolism: A Scientific Statement From the American Heart Association. Circulation research. 2016;118(10):1659-1701.
4. Eelen G, de Zeeuw P, Simons M, Carmeliet P. Endothelial cell metabolism in normal and diseased vasculature. Circulation research. 2015;116(7):1231-1244.
5. Gomez-Arroyo J, Mizuno S, Szczepanek K, Van Tassell B, Natarajan R, dos Remedios CG, Drake JI, et al. Metabolic gene remodeling and mitochondrial dysfunction in failing right ventricular hypertrophy secondary to pulmonary arterial hypertension. Circ Heart Fail. 2013;6(1):136-144.
6. Ryan J, Dasgupta A, Huston J, Chen KH, Archer SL. Mitochondrial dynamics in pulmonary arterial hypertension. Journal of molecular medicine. 2015;93(3):229-242.
7. Obre E, Rossignol R. Emerging concepts in bioenergetics and cancer research: metabolic flexibility, coupling, symbiosis, switch, oxidative tumors, metabolic remodeling, signaling and bioenergetic therapy. Int J Biochem Cell Biol. 2015;59:167-181.
8. Chen X, Talati M, Fessel JP, Hemnes AR, Gladson S, French J, Shay S, et al. Estrogen Metabolite 16alpha-Hydroxyestrone Exacerbates Bone Morphogenetic Protein Receptor Type II-Associated Pulmonary Arterial Hypertension Through MicroRNA-29-Mediated Modulation of Cellular Metabolism. Circulation. 2016;133(1):82-97.
9. Hemnes AR, Brittain EL, Trammell AW, Fessel JP, Austin ED, Penner N, Maynard KB, et al. Evidence for right ventricular lipotoxicity in heritable pulmonary arterial hypertension. Am J Respir Crit Care Med. 2014;189(3):325-334.
10. West J, Niswender KD, Johnson JA, Pugh ME, Gleaves L, Fessel JP, Hemnes AR. A potential role for insulin resistance in experimental pulmonary hypertension. The European respiratory journal. 2013;41(4):861-871.
11. Dyck JR, Hopkins TA, Bonnet S, Michelakis ED, Young ME, Watanabe M, Kawase Y, Jishage K, Lopaschuk GD. Absence of malonyl coenzyme A decarboxylase in mice increases cardiac glucose oxidation and protects the heart from ischemic injury. Circulation. 2006;114(16):1721-1728.
12. Talati MH, Brittain EL, Fessel JP, Penner N, Atkinson J, Funke M, Grueter C, et al. Mechanisms of Lipid Accumulation in the Bone Morphogenic Protein Receptor 2 Mutant Right Ventricle. Am J Respir Crit Care Med. 2016.
13. Brittain EL, Talati M, Fessel JP, Zhu H, Penner N, Calcutt MW, West JD, et al. Fatty Acid Metabolic Defects and Right Ventricular Lipotoxicity in Human Pulmonary Arterial Hypertension. Circulation. 2016;133(20):1936-1944.
14. Piao L, Fang YH, Parikh K, Ryan JJ, Toth PT, Archer SL. Cardiac glutaminolysis: a maladaptive cancer metabolism pathway in the right ventricle in pulmonary hypertension. Journal of molecular medicine. 2013;91(10):1185-1197.
15. Li C, Zhang G, Zhao L, Ma Z, Chen H. Metabolic reprogramming in cancer cells: glycolysis, glutaminolysis, and Bcl-2 proteins as novel therapeutic targets for cancer. World J Surg Oncol. 2016;14(1):15.
16. Yang C, Ko B, Hensley CT, Jiang L, Wasti AT, Kim J, Sudderth J, et al. Glutamine oxidation maintains the TCA cycle and cell survival during impaired mitochondrial pyruvate transport. Molecular cell. 2014;56(3):414-424.
17. Jiang L, Shestov AA, Swain P, Yang C, Parker SJ, Wang QA, Terada LS, et al. Reductive carboxylation supports redox homeostasis during anchorage-independent growth. Nature. 2016;532(7598):255-258.
18. DeBerardinis RJ, Chandel NS. Fundamentals of cancer metabolism. Sci Adv. 2016;2(5): e 1600200.
19. Kirabo A, Fontana V, de Faria AP, Loperena R, Galindo CL, Wu J, Bikineyeva AT, et al. DC isoketal-modified proteins activate T cells and promote hypertension. The Journal of clinical investigation. 2014;124(10):4642-4656.
20. Majka S, Hagen M, Blackwell T, Harral J, Johnson JA, Gendron R, Paradis H, et al. Physiologic and molecular consequences of endothelial Bmpr2 mutation. Respir Res. 2011;12:84.
21. Greene J, Henderson JW, Wikswo JP. Rapid and precise determination of cellular amino acid flux rates using HPLC with automated derivatization with absorbance detection. Agilent Application Notes. 2009.
22. Murphy TA, Young JD. ETA: robust software for determination of cell specific rates from extracellular time courses. Biotechnol Bioeng. 2013;110(6):1748-1758.
23. Shah AT, Demory Beckler M, Walsh AJ, Jones WP, Pohlmann PR, Skala MC. Optical metabolic imaging of treatment response in human head and neck squamous cell carcinoma. PLoS One. 2014;9(3):e90746.
24. Fessel JP, Flynn CR, Robinson LJ, Penner NL, Gladson S, Kang CJ, Wasserman DH, Hemnes AR, West JD. Hyperoxia synergizes with mutant bone morphogenic protein receptor 2 to cause metabolic stress, oxidant injury, and pulmonary hypertension. American journal of respiratory cell and molecular biology. 2013;49(5):778-787.
25. Johnson JA, Hemnes AR, Perrien DS, Schuster M, Robinson LJ, Gladson S, Loibner H, et al. Cytoskeletal defects in Bmpr2-associated pulmonary arterial hypertension. American journal of physiology. Lung cellular and molecular physiology. 2012;302(5):L474-484.
26. Fares WH, Ford HJ, Ghio AJ, Aris RM. Safety and feasibility of obtaining wedged pulmonary artery samples and differential distribution of biomarkers in pulmonary hypertension. Pulmonary circulation. 2012;2(4):477-482.
27. Monahan K, Scott TA, Su YR, Lenneman CG, Zhao DX, Robbins IM, Hemnes AR. Reproducibility of intracardiac and transpulmonary biomarkers in the evaluation of pulmonary hypertension. Pulmonary circulation. 2013;3(2):345-349.
28. Skala MC, Riching KM, Gendron-Fitzpatrick A, Eickhoff J, Eliceiri KW, White JG, Ramanuj am N. In vivo multiphoton microscopy of NADH and FAD redox states, fluorescence lifetimes, and cellular morphology in precancerous epithelia. Proceedings of the National Academy of Sciences of the United States of America. 2007;104(49):19494-19499.
29. Chance B, Schoener B, Oshino R, Itshak F, Nakase Y. Oxidation-reduction ratio studies of mitochondria in freeze-trapped samples. NADH and flavoprotein fluorescence signals. The Journal of biological chemistry. 1979;254(11):4764-4771.
30. Barlow CH, Harden WR, 3rd, Harken AH, Simson MB, Haselgrove JC, Chance B, O'Connor M, Austin G. Fluorescence mapping of mitochondrial redox changes in heart and brain. Crit Care Med. 1979;7(9):402-406.
31. Sanchez EL, Lagunoff M. Viral activation of cellular metabolism. Virology. 2015;479-480:609-618.
32. Hough KP, Chisolm DA, Weinmann AS. Transcriptional regulation of T cell metabolism. Mol Immunol. 2015;68(2 Pt C):520-526.
33. Courtnay R, Ngo DC, Malik N, Ververis K, Tortorella SM, Karagiannis TC. Cancer metabolism and the Warburg effect: the role of HIF-1 and PI3K. Mol Biol Rep. 2015;42(4):841-851.
34. Ryan JJ, Archer SL. The right ventricle in pulmonary arterial hypertension: disorders of metabolism, angiogenesis and adrenergic signaling in right ventricular failure. Circulation research. 2014;115(1):176-188.
35. Chen JQ, Russo J. Dysregulation of glucose transport, glycolysis, TCA cycle and glutaminolysis by oncogenes and tumor suppressors in cancer cells. Biochim Biophys Acta. 2012;1826(2):370-384.
36. Tuder RM, Archer SL, Dorfmuller P, Erzurum SC, Guignabert C, Michelakis E, Rabinovitch M, Schermuly R, Stenmark KR, Morrell NW. Relevant issues in the pathology and pathobiology of pulmonary hypertension. J Am Coll Cardiol. 2013;62(25 Suppl):D4-12.
37. Pisarcik S, Maylor J, Lu W, Yun X, Undem C, Sylvester JT, Semenza GL, Shimoda LA. Activation of hypoxia-inducible factor-1 in pulmonary arterial smooth muscle cells by endothelin-1. American journal of physiology. Lung cellular and molecular physiology. 2013;304(8):L549-561.
38. Fijalkowska I, Xu W, Comhair SA, Janocha AJ, Mavrakis LA, Krishnamachary B, Zhen L, et al. Hypoxia inducible-factor1alpha regulates the metabolic shift of pulmonary hypertensive endothelial cells. The American journal of pathology. 2010;176(3):1130-1138.
39. Wise DR, Ward PS, Shay JE, Cross JR, Gruber JJ, Sachdeva UM, Platt JM, DeMatteo RG, Simon MC, Thompson CB. Hypoxia promotes isocitrate dehydrogenase-dependent carboxylation of alpha-ketoglutarate to citrate to support cell growth and viability. Proceedings of the National Academy of Sciences of the United States of America. 2011;108(49):19611-19616.
40. Wise DR, DeBerardinis RJ, Mancuso A, Sayed N, Zhang XY, Pfeiffer HK, Nissim I, et al. Myc regulates a transcriptional program that stimulates mitochondrial glutaminolysis and leads to glutamine addiction. Proceedings of the National Academy of Sciences of the United States of America. 2008;105(48):18782-18787.
41. Hosios AM, Hecht VC, Danai LV, Johnson MO, Rathmell JC, Steinhauser ML, Manalis SR, Vander Heiden MG. Amino Acids Rather than Glucose Account for the Majority of Cell Mass in Proliferating Mammalian Cells. Dev Cell. 2016;36(5):540-549.
42. Haigis MC, Deng CX, Finley LW, Kim HS, Gius D. SIRT3 is a mitochondrial tumor suppressor: a scientific tale that connects aberrant cellular ROS, the Warburg effect, and carcinogenesis. Cancer Res. 2012;72(10):2468-2472.
43. Finley LW, Carracedo A, Lee J, Souza A, Egia A, Zhang J, Teruya-Feldstein J, et al. SIRT3 opposes reprogramming of cancer cell metabolism through HIF1alpha destabilization. Cancer cell. 2011;19(3):416-428.
44. Paulin R, Dromparis P, Sutendra G, Gurtu V, Zervopoulos S, Bowers L, Haromy A, et al. Sirtuin 3 deficiency is associated with inhibited mitochondrial function and pulmonary arterial hypertension in rodents and humans. Cell metabolism. 2014;20(5):827-839.
45. Lane KL, Talati M, Austin E, Hemnes AR, Johnson JA, Fessel JP, Blackwell T, et al. Oxidative injury is a common consequence of BMPR2 mutations. Pulmonary circulation. 2011;1(1):72-83.
46. Diebold I, Hennigs JK, Miyagawa K, Li CG, Nickel NP, Kaschwich M, Cao A, et al. BMPR2 Preserves Mitochondrial Function and DNA during Reoxygenation to Promote Endothelial Cell Survival and Reverse Pulmonary Hypertension. Cell metabolism. 2015;21(4):596-608.
47. Fessel JP, Hamid R, Wittmann BM, Robinson LJ, Blackwell T, Tada Y, Tanabe N, Tatsumi K, Hemnes AR, West JD. Metabolomic analysis of bone morphogenetic protein receptor type 2 mutations in human pulmonary endothelium reveals widespread metabolic reprogramming. Pulmonary circulation. 2012;2(2):201-213.
48. Xu W, Koeck T, Lara AR, Neumann D, DiFilippo FP, Koo M, Janocha AJ, et al. Alterations of cellular bioenergetics in pulmonary artery endothelial cells. Proceedings of the National Academy of Sciences of the United States of America. 2007;104(4):1342-1347.
49. Lai YC, Tabima DM, Dube JJ, Hughan KS, Vanderpool RR, Goncharov DA, St Croix CM, et al. SIRT3-AMP-Activated Protein Kinase Activation by Nitrite and Metformin Improves Hyperglycemia and Normalizes Pulmonary Hypertension Associated With Heart Failure With Preserved Ejection Fraction. Circulation. 2016;133(8):717-731.
50. Waypa GB, Osborne SW, Marks JD, Berkelhamer SK, Kondapalli J, Schumacker PT. Sirtuin 3 deficiency does not augment hypoxia-induced pulmonary hypertension. American journal of respiratory cell and molecular biology. 2013;49(6):885-891.
51. Nguyen TT, Caito SW, Zackert WE, West JD, Zhu S, Aschner M, Fessel JP, Roberts LJ, 2nd. Scavengers of reactive gamma-ketoaldehydes extend Caenorhabditis elegans lifespan and healthspan through protein-level interactions with SIR-2.1 and ETS-7. Aging (Albany NY). 2016.
52. Bertero T, Oldham WM, Cottrill KA, Pisano S, Vanderpool RR, Yu Q, Zhao J, et al. Vascular stiffness mechanoactivates YAP/TAZ-dependent glutaminolysis to drive pulmonary hypertension. The Journal of clinical investigation. 2016.
53. Schoors S, Bruning U, Missiaen R, Queiroz KC, Borgers G, Elia I, Zecchin A, et al. Fatty acid carbon is essential for dNTP synthesis in endothelial cells. Nature. 2015;520(7546):192-197.

## Claims

1. A compound for use in a method of treating or preventing WHO group I pulmonary hypertension, wherein the compound is of the following formula: 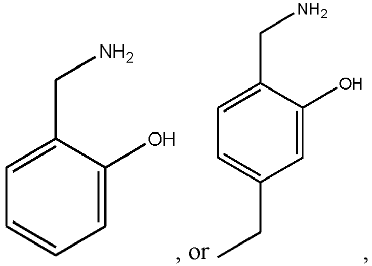 or a pharmaceutically acceptable salt thereof.

2. A compound for use according to claim 1, wherein the compound is of the following formula: 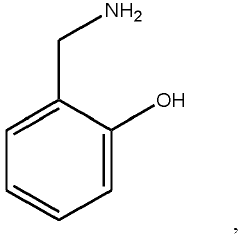 or a pharmaceutically acceptable salt thereof.

3. A compound for use according to any one of the preceding claims in a method of treating or preventing heritable pulmonary hypertension.

## Patentansprüche

1. Verbindung zur Verwendung in einem Verfahren zur Behandlung oder Prävention von pulmonaler Hypertonie der WHO-Gruppe I, wobei die Verbindung die folgende Formel aufweist: 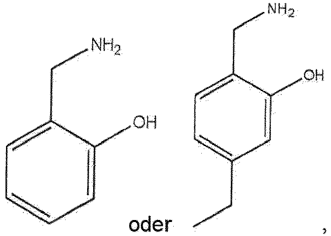 oder ein pharmazeutisch annehmbares Salz davon.

2. Verbindung zur Verwendung nach Anspruch 1, wobei die Verbindung die folgende Formel aufweist: 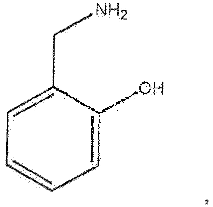 oder ein pharmazeutisch annehmbares Salz davon.

3. Verbindung zur Verwendung nach einem der vorangegangenen Ansprüche in einem Verfahren zur Behandlung oder Prävention von hereditärer pulmonaler Hypertonie.

## Revendications

1. Composé à utiliser dans un procédé de traitement ou de prévention de l'hypertension pulmonaire du groupe I de l'OMS, dans lequel le composé répond à la formule suivante : 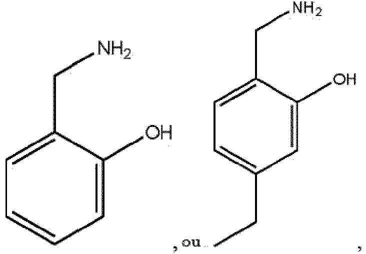 ou un sel pharmaceutiquement acceptable de celui-ci.

2. Composé selon la revendication 1, dans lequel le composé répond à la formule suivante : 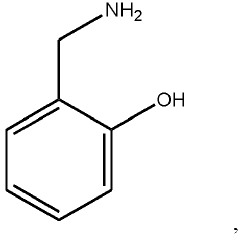 ou un sel pharmaceutiquement acceptable de celui-ci.

3. Composé à utiliser selon l'une quelconque des revendications précédentes dans un procédé de traitement ou de prévention de l'hypertension pulmonaire héréditaire.
